# EUROPEAN PATENT SPECIFICATION

(11) **EP 2 379 015 B1**
(45) Date of publication and mention of the grant of the patent: **26.02.2020**
(21) Application number: 09760554.7
(22) Date of filing: 05.11.2009
(51) Int. Cl.: A61F 2/36, A61F 2/40

(54) **MEDICAL DEVICE**
MEDIZINISCHE VORRICHTUNG
DISPOSITIF MÉDICAL

(30) Priority: 05.11.2008 GB 0820233
(43) Date of publication of application: 26.10.2011
(73) Proprietor: T.J. Smith & Nephew Limited, Hull HU3 2BN (GB)
(72) Inventor: BRUCE, Warwick, J., M., Lewisham, NSW 2049 (AU)
(74) Representative: Smith & Nephew
(86) International application number: PCT/GB2009/002611
(87) International publication number: WO 2010/052461

(56) References cited:
- EP-A- 1 285 639
- WO-A-2007/026003
- DE-B3-102007 032 583
- US-A- 4 728 330
- US-A- 6 033 439
- US-A- 6 129 764

## Description

The present invention relates to medical devices, particularly hip implants.

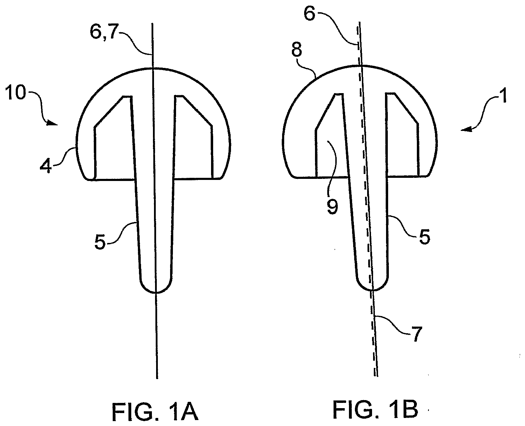

Known femoral head resurfacing designs have the stem axis and the femoral head axis concurrent. That is, the stem axis and the femoral head axis are co-linear (see Figure 1A). Femoral anatomy varies significantly between patients. The centre of the femoral neck is often not the centre of the femoral head. Consequently, mismatches can occur when a femoral head is resurfaced with a conventional resurfacing design. Such mismatches mean that known resurfacing designs do not reproduce the natural biomechanics and stability of the joint. This can lead to many problems for the patient, ranging from discomfort to pain and ultimately to failure, necessitating revision surgery.

Patent document DE102007032583 discloses a surface replacement for the natural sliding or articulating surfaces of the acetabulum and the hip-joint head by an offset-resurfacing hip-joint implant.

With known femoral head resurfacing designs there is also little intra-operative flexibility in positioning the centre of the femoral head once the stem has been prepared. Consequently, the mismatches cannot be rectified and the above problems, are encountered.

According to an aspect there is provided an eccentric resurfacing head.

According to an aspect there is provided an implant, comprising: a head having a central axis; and a stem having a stem axis, wherein the head central axis and the stem axis are not co-linear.

According to an aspect there is provided an implant for resurfacing a femoral head, comprising: a head having a central axis; and a stem having a stem axis, wherein the head central axis and the stem axis are not co-linear.

According to an aspect of the present invention, there is provided an implant, comprising: a head having a bearing surface centre; and a stem having a stem axis, wherein the stem axis is offset compared to the head bearing surface centre.

According to an aspect of the present invention, there is provided an implant for resurfacing a femoral head, comprising: a head having a bearing surface centre; and a stem having a stem axis, wherein the stem axis is offset compared to the head bearing surface centre.

According to an aspect of the present invention, there is provided a resurfacing femoral head and stem, wherein the stem axis is offset compared to the bearing surface centre.

According to an aspect of the present invention, there is provided an implant comprising a head having a bearing surface and a recess inside the head with a stem, the stem and recess having an axis, wherein the stem/recess axis and the head axis are offset from each other.

According to an aspect of the present invention, there is provided an implant for resurfacing a femoral head, comprising a head having a bearing surface and a recess inside the head with a stem, the stem and recess having an axis, wherein the stem/recess axis and the head axis are offset from each other.

According to an aspect of the present invention, there is provided an implant, comprising: a head having an external surface defining a bearing surface, the bearing surface having a centre, and an internal surface defining a recess within the head; and a stem disposed on the internal surface of the head and having a stem axis, wherein the stem axis is offset compared to the head bearing surface centre.

According to an aspect of the present invention, there is provided an implant for resurfacing a femoral head, comprising: a head having an external surface defining a bearing surface, the bearing surface having a centre, and an internal surface defining a recess within the head; and a stem disposed on the internal surface of the head and having a stem axis, wherein the stem axis is offset compared to the head bearing surface centre.

According to an aspect there is provided an implant, comprising: a head having an external surface defining a bearing surface, the bearing surface having a centre, and an internal surface defining a recess within the head; and a stem disposed on the internal surface of the head and having a stem axis, wherein the stem axis is offset compared to the head bearing surface centre, and wherein the stem and the internal surface are concentric.

According to an aspect there is provided an implant for resurfacing a femoral head, comprising: a head having an external surface defining a bearing surface, the bearing surface having a centre, and an internal surface defining a recess within the head; and a stem disposed on the internal surface of the head and having a stem axis, wherein the stem axis is offset compared to the head bearing surface centre, and wherein the stem and the internal surface are concentric.

The offset between the stem axis and the head bearing surface centre may be any suitable amount. The offset may be an integer or non-integer amount. The offset may be in the range 1-10 mm. The offset may be in the range 1-5 mm. The offset may be in the range 1-3 mm. The offset may be around 1 mm. The offset may be around 2 mm. The offset may be around 3 mm.

Preferably, the internal geometry of the head remains symmetric. This may be achieved by thickening one wall of the head (see Figure 1B). This allows the surgeon to prepare the bone and then decide where to position the centre of the bearing.

The stem axis may be parallel, or substantially parallel, to the head centre axis.

The stem may be angled. That is, the stem axis may be angled with respect to the head centre axis.

Preferably, the implant is a monobloc/single part comprising a head and a stem.

An advantage associated with monobloc embodiments of the present invention is that they are mechanically superior to two-component embodiments. For example, a two component device has fretting interfaces which can lead to wear. In contrast, a single component device does not have fretting interfaces and is therefore more resistant to wear.

Implants according to the present invention may comprise one or more bone ingrowth surfaces.

Implants according to the present invention may be made of any suitable material. The implant may comprise metal. The implant may comprise metal alloy. The implant may comprise stainless steel. The implant may comprise titanium. The implant may comprise cobalt chrome.

Also disclosed herein but not claimed is a method of implantation, comprising the steps of: providing an implant according to the present invention; and implanting the implant into a body.

Not part of the invention is a method for resurfacing a femoral head, comprising the steps of: providing an implant according to the present invention; and implanting the implant with reference to the centre of the femoral neck.

An advantage associated with referencing the centre of the neck of the femur is that the risk of neck fracture is minimised or eliminated.

In use, the implant may be cemented.

In use, the implant may not be cemented.

Implants according to the present invention allow the surgeon final control on the bearing centre of the hip. This is important in restoring the natural biomechanics and stability of the joint.

Reference will now be made, by way of example, to the accompanying drawings, in which:
Figures 1A and 1B show cross-sections of a known femoral head resurfacing implant and a femoral head resurfacing implant according to an embodiment of the present invention, respectively;
Figures 2A and 2B show a top plan view and a cross-section view of a femoral head resurfacing implant according to another embodiment of the present invention, respectively;
Figures 3A and 3B show a top plan view and a cross-section view of a femoral head resurfacing implant according to another embodiment of the present invention, respectively; and
Figures 4A, 4B and 4C show cross-sections of a known femoral head resurfacing implant, the embodiment shown in Figure 2B and the embodiment shown in Figure 3B, respectively.

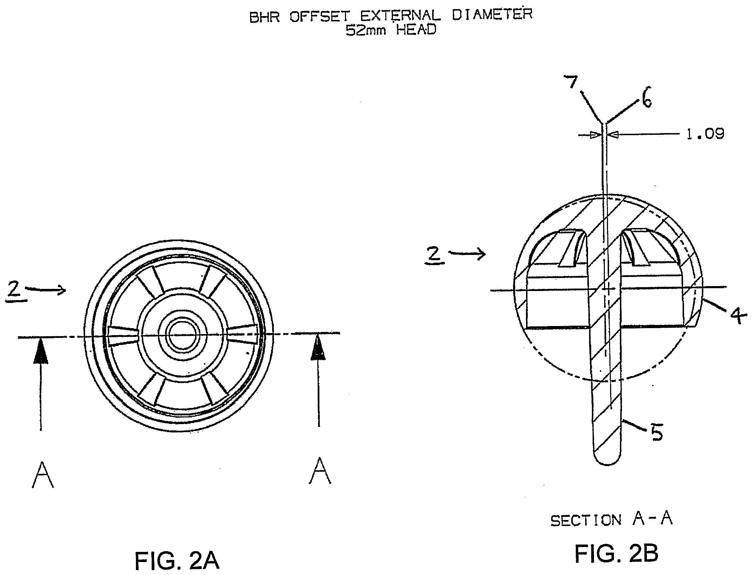

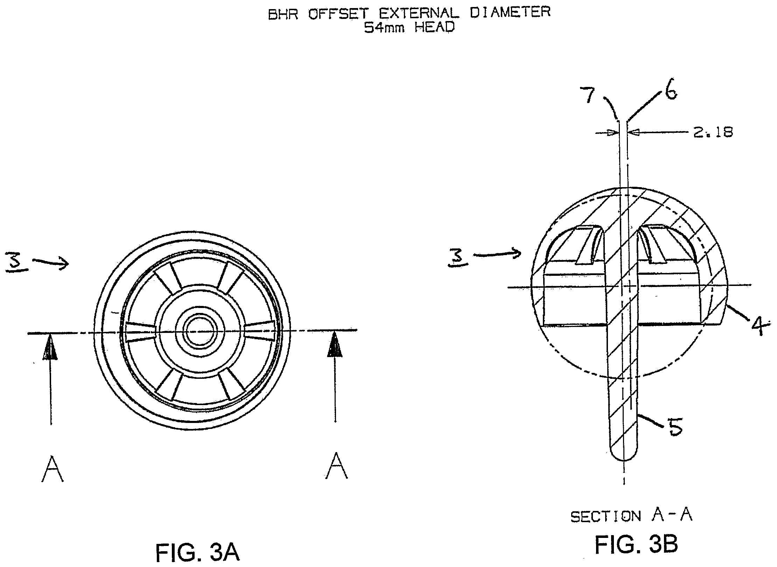

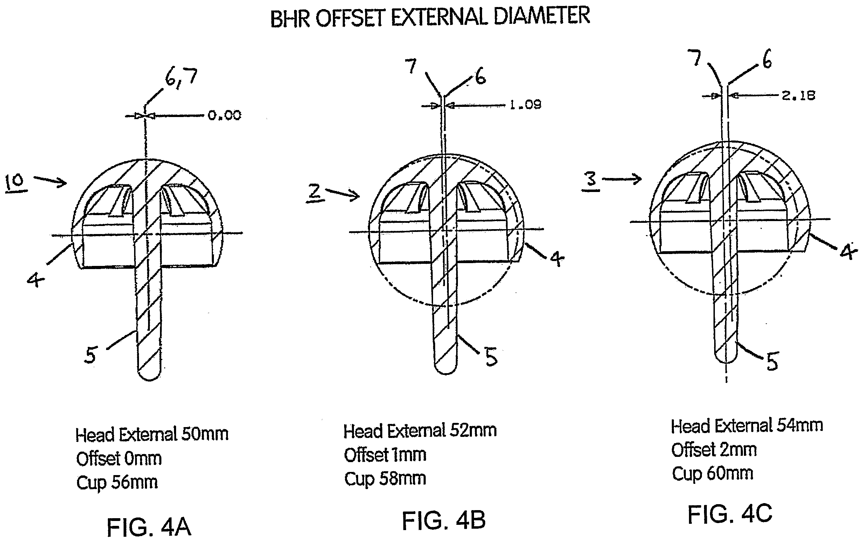

As shown in Figures 1A and 4A, for known femoral head resurfacing implants (10) the femoral head axis (6) and the stem axis (7) are co-linear. That is, the stem (5) is concentric to the head (4) centre. As shown in Figures 1B, 2A, 2B, 3A, 3B, 4B and 4C, for femoral head resurfacing implants (1-3) in accordance with the present invention, the femoral head axis (6) and the stem axis (7) are offset such that they are not co-linear. As shown in Figure 1B, implants (1) according to an embodiment of the present invention comprise a stem (5) that is eccentric to the articulating surface (8) but concentric to the internal profile (9). Figures 2A, 2B and 4B show an implant (2) having an offset of approximately 1 mm. Figures 3A, 3B and 4C show an implant (3) having an offset of approximately 2 mm.

For example, the embodiment shown in Figure 4A may have a head external diameter of around 50 mm, an offset of 0 mm, and be coupled with a cup having a diameter of around 56 mm. The embodiment shown in Figure 4B may have a head external diameter of around 52 mm, an offset of around 1 mm, and be coupled with a cup having a diameter of around 58 mm. The embodiment shown in Figure 4C may have a head external diameter of around 54 mm, an offset of around 2 mm, and be coupled with a cup having a diameter of around 60 mm.

## Claims

1. A femoral implant (2,3) for resurfacing a femoral head comprising:
a head (4) comprising an external bearing surface having a centre and defining a head axis (6) passing through the centre, and an internal surface defining a recess within the head and a recess axis; and
a stem (5) extending from the internal surface of the head and having a stem axis (7),
**characterised in that** the stem (5) and the head (4) are integral to provide a one-piece implant, and wherein the stem axis (7) is offset
compared to the head bearing surface centre
and parallel to the head axis (6) and co-axial to the recess axis such that the internal surface of the head remains symmetric in use, and wherein the stem (5) extends downwardly beyond the external bearing surface of the head.

2. An implant according to claim 1, wherein the offset is in the range 1-3 mm.

## Patentansprüche

1. Ein Femurimplantat (2, 3) für einen Oberflächenersatz eines Femurkopfes, beinhaltend:
einen Kopf (4), der eine äußere Auflagefläche, die eine Mitte aufweist und eine Kopfachse (6) definiert, welche durch die Mitte verläuft, und eine innere Oberfläche, die eine Aussparung innerhalb des Kopfes und eine Aussparungsachse definiert,
beinhaltet; und
einen Schaft (5), der sich von der inneren Oberfläche des Kopfes erstreckt und eine Schaftachse (7) aufweist,
**dadurch gekennzeichnet, dass** der Schaft (5) und der Kopf (4) integral sind, um ein einstückiges Implantat bereitzustellen, und wobei die Schaftachse (7) im Vergleich zu der Mitte der Kopfauflagefläche versetzt und parallel zu der Kopfachse (6) und koaxial zu der Aussparungsachse ist, sodass die innere Oberfläche des Kopfes im Gebrauch symmetrisch bleibt, und wobei sich der Schaft (5) über die äußere Auflagefläche des Kopfes hinaus abwärts erstreckt.

2. Implantat gemäß Anspruch 1, wobei der Versatz in dem Bereich von 1-3 mm liegt.

## Revendications

1. Un implant fémoral (2, 3) pour le resurfaçage d'une tête fémorale comprenant :
une tête (4) comprenant une surface d'appui extérieure ayant un centre et définissant un axe de tête (6) passant par le centre, et une surface intérieure définissant un renfoncement au sein de la tête et un axe de renfoncement ; et
une tige (5) s'étendant depuis la surface intérieure de la tête et ayant un axe de tige (7),
**caractérisé en ce que** la tige (5) et la tête (4) sont solidaires afin de fournir un implant monobloc, et dans lequel l'axe de tige (7) est décalé par comparaison au centre de surface d'appui de tête et parallèle à l'axe de tête (6) et coaxial à l'axe de renfoncement de telle sorte que la surface intérieure de la tête demeure symétrique lors de l'utilisation, et dans lequel la tige (5) s'étend vers le bas au-delà de la surface d'appui extérieure de la tête.

2. Un implant selon la revendication 1, dans lequel le décalage est compris dans l'intervalle de 1 à 3 mm.
